(19) Europäisches Patentamt
European Patent Office
Office européen des brevets

(11) Veröffentlichungsnummer: **0 000 895**
**A1**

(12) **EUROPÄISCHE PATENTANMELDUNG**

(21) Anmeldenummer: **78100609.3**

(22) Anmeldetag: **07.08.78**

(51) Int. Cl.2: **C 07 C 161/00**, A 01 N 9/12, C 07 C 149/437, C 07 D 295/22

(30) Priorität: **20.08.77 DE 2737606**

(43) Veröffentlichungstag der Anmeldung: **07.03.79 Patentblatt 79/5**

(84) Benannte Vertragsstaaten: **BE CH DE FR GB NL**

(71) Anmelder: **Bayer Aktiengesellschaft, Zentralbereich Patente,Marken und Lizenzen Bayerwerk, D-5090 Leverkusen 1 (DE)**

(72) Erfinder: **Hoffmann, Hellmut, Prof. Dr., Tersteegenweg 17, D-5600 Wuppertal 1 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

(54) **Aryl-N-alkyl-carbamate, Verfahren zu ihrer Herstellung und ihre Verwendung als Insektizide.**

(57) Es werden Aryl-N-alkylcarbamate der Formel

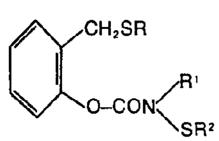

synthetisiert, worin R und R' für gleiches oder verschiedenes Alkyl und $R^2$ für Dialkylamino oder einen stickstoffhaltigen Heterocyclus stehen, wobei der Heterocyclus noch weitere Heteroatome enthalten kann.

Diese Verbindungen zeichnen sich durch starke insektizide Eigenschaften aus.

Sie werden erhalten, wenn man Aryl-N-alkylcarbamate der Formel

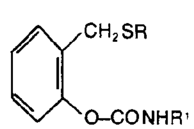

in welcher R und R' die oben angegebene Bedeutung haben, mit Sulfenylhalogeniden der Formel $R^2$-S-Hal, gegebenenfalls in Gegenwart eines Säureakzeptors und eines Lösungsmittels umsetzt.

EP 0 000 895 A1

0000895

BAYER AKTIENGESELLSCHAFT
Zentralbereich
Patente, Marken und Lizenzen

5090 Leverkusen, Bayerwerk
Rt-by

Typ Ia

# Aryl-N-alkyl-carbamate, Verfahren zu ihrer Herstellung und ihre Verwendung als Insektizide

Die vorliegende Erfindung betrifft neue Aryl-N-alkyl-carbamate, ein Verfahren zu ihrer Herstellung und ihre Verwendung als Insektizide.

Es ist bereits bekannt, daß N-Methyl-phenylcarbamate, z.B. 2-sek.-Butylphenyl- bzw. 2-iso-Propoxyphenyl-N-methylcarbamat, insektizide Eigenschaften aufweisen (vergleiche US-Patent 3 111 539, Deutsche Auslegeschriften 1 159 929 und 1 166 18o).

Es wurden nun die neuen Aryl-N-alkyl-carbamate der Formel

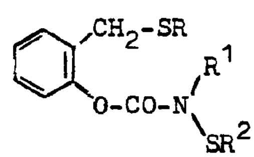

(I)

synthetisiert,

worin
R und $R^1$ für gleiches oder verschiedenes Alkyl und

$R^2$ für Dialkylamino oder einen stickstoffhaltigen Heterocyclus stehen, wobei der Heterocyclus noch weitere Heteroatome enthalten kann.

Diese neuen Verbindungen zeichnen sich durch starke insektizide Eigenschaften aus.

Weiterhin wurde gefunden, daß die Aryl-N-alkyl-carbamate der Formel (I) erhalten werden, wenn man Aryl-N-alkyl-carbamate der Formel (II)

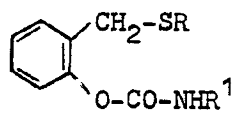

(II)

in welcher

R und $R^1$ die oben angegebene Bedeutung haben,

mit Sulfenylhalogeniden der Formel (III)

$$R^2\text{-S-Hal} \qquad \text{(III)}$$

in welcher

$R^2$ die oben angegebene Bedeutung hat und

Hal für Halogen, vorzugsweise Chlor, steht,

gegebenenfalls in Gegenwart eines Säureakzeptors und eines Lösungsmittels umsetzt.

Überraschenderweise besitzen die erfindungsgemäßen Aryl-N-alkyl-carbamate eine bessere insektizide Wirkung als die aus der Literatur vorbekannten Carbamate analoger Konstitution und gleicher Wirkungsrichtung. Die Stoffe gemäß vorliegender Erfindung stellen somit eine echte Bereicherung der Technik dar.

Verwendet man beispielsweise 2-n-Propylthiomethyl-phenyl-N-n-propyl-carbamat und Diäthylaminosulfenylchlorid als Ausgangsstoffe, so kann der Reaktionsverlauf durch das folgende Formelschema wiedergegeben werden:

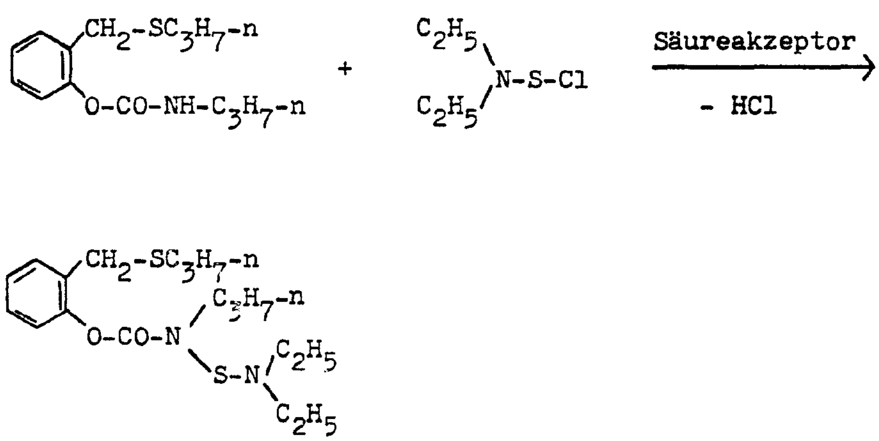


Die zu verwendenden Ausgangsstoffe sind durch die Formeln (II) und (III) allgemein definiert. Vorzugsweise stehen darin jedoch

R und $R^1$ für gleiches oder verschiedenes, geradkettiges oder verzweigtes Alkyl mit 1 bis 6, vorzugsweise 1 bis 4, Kohlenstoffatomen und

$R^2$ für Dialkylamino mit 1 bis 4, vorzugsweise 1 bis 3, Kohlenstoffatomen je Alkylrest, für einen 5 bis 7 gliedrigen, vorzugsweise 5 oder 6 gliedrigen, gesättigten Stickstoffheterocyclus, der gegebenenfalls durch Sauerstoff, Schwefel oder einem weiteren Stickstoffatomen, bevorzugt durch Sauerstoff, unterbrochen sein kann.

Die als Ausgangsstoffe zu verwendenden Aryl-N-alkyl-carbamate (II) sind bekannt oder können nach bekannten Verfahren leicht hergestellt werden (vergleiche Deutsche Auslegeschrift 1 254 617 und Belgische Patentschrift 746 649). Als Beispiele dafür seien im einzelnen genannt:
2-Methylthiomethyl-phenyl-, 2-Äthylthiomethyl-phenyl-, 2-n-Propylthiomethyl-phenyl- und 2-iso-Propylthiomethyl-phenyl-, 2-n-Butylthiomethyl-phenyl-, 2-iso-Butylthiomethyl-phenyl- und 2-sek.-Butylthiomethyl-phenyl-N-methyl- bzw. -N-äthyl-, -N-n-propyl-, -N-iso-propyl-, -N-n-butyl-, -N-iso-butyl- und -N-sek.-butyl-carbamat.

Die weiterhin als Ausgangsstoffe zu verwendenden Sulfenylhalogenide (III) sind ebenfalls bekannt oder nach bekannten Verfahren herstellbar (vergleiche Belgische Patentschriften 817 515 und 817 517). Als Beispiele dafür seien im einzelnen genannt:
Dimethylamino-, Diäthylamino-, Di-n-propylamino-, Di-isopropylamino-, Pyrrolidinyl(1), Piperidyl(1) und Morpholinyl(4)-sulfenylchlorid.

Das Verfahren zur Herstellung der erfindungsgemäßen Verbindungen wird bevorzugt unter Mitverwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol, oder Äther, z.B. Diäthyl- und Dibutyläther, Dioxan, ferner Ketone, beispielsweise Aceton, Methyläthyl-, Methylisopropyl- und Methylisobutylketon, außerdem Nitrile, wie Aceto- und Propionitril oder Pyridin.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 80°C, vorzugsweise bei 10 bis 35°C.

Die Umsetzung läßt man im allgemeinen bei Normaldruck ablaufen.

Zur Durchführung des Verfahrens setzt man die Ausgangskomponenten meist in äquivalentem Verhältnis ein. Ein Überschuß der einen oder anderen Reaktionskomponente bringt keine wesentlichen Vorteile. Die Reaktionskomponenten werden meist in einem der oben angeführten Lösungsmittel zusammengegeben und meist bei Raumtemperatur zur Vervollständigung der Reaktion mehrere Stunden gerührt. Danach gibt man die Reaktionsmischung auf ein Gemisch aus Eis/Säure und nimmt das sich ausscheidende Öl in einem organischen Lösungsmittel, z.B. Toluol, auf. Die organische Phase wird wie üblich durch Waschen, Trocknen und Abdestillieren des Lösungsmittels aufgearbeitet.

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa

decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.
Diese Formulierungen werden in bekannter Weise hergestellt, z.B. durch Vermischen der Wirkstoffe mit Streckmitteln, also

flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat,

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Myzus-Test (Kontakt-Wirkung)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Kohlpflanzen (Brassica oleracea), welche stark von der Pfirsichblattlaus (Myzus persicae) befallen sind, tropfnaß besprüht.

Bei diesem Test zeigen z.B. die folgenden Verbindungen der Herstellungsbeispiele überlegene Wirkung gegenüber dem Stand der Technik, 1, 10, 6, 3, 12, 2, 8, 4, 7, 11, 5

Beispiel B

Grenzkonzentrations-Test / Wurzelsystemische Wirkung

Testinsekt: Myzus persicae

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere.

Bei diesem Test zeigen z.B. die folgenden Verbindungen der Herstellungsbeispiele überlegene Wirkung gegenüber dem Stand der Technik: 1, 2.

Beispiel C

Test mit parasitierenden Fliegenlarven

Testinsekt: Lucilia cuprina

Emulgator: 80 Gew.-Teile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration.

Etwa 20 Fliegenlarven (Lucilia cuprina) werden in ein mit Wattestopfen entsprechender Größe beschicktes Teströhrchen gebracht, welches ca. 3 ml einer 20 %igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 2, 3, 7, 8.

Herstellungsbeispiele

Beispiel 1:

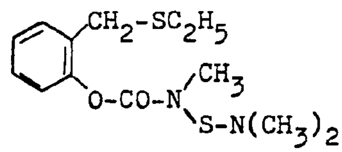

Eine Lösung aus 113 g (o,5 Mol) 2-Äthylthiomethylphenyl-N-methyl-carbamat in 3oo ml Pyridin wird bei Raumtemperatur und unter Feuchtigkeitsausschluß mit 57 g (o,51 Mol) Dimethylaminosulfenylchlorid versetzt und 18 Stunden bei Raumtemperatur gerührt. Das Reaktionsgemisch wird auf eine Mischung aus Eis und 36o ml reiner, konzentrierter Salzsäure gegossen und das ausgefallene Öl in Toluol aufgenommen. Die organische Phase wird mit Wasser, anschließend mit einer Bicarbonatlösung gewaschen und über Natriumsulfat getrocknet. Das Toluol wird im Vakuum am Rotationsverdampfer abgezogen und der verbleibende Rückstand im Hochvakuum bei einer Badtemperatur von 5o-6o°C andestilliert. Man erhält 9o g (6o % der Theorie) 2-Äthylthiomethyl-phenyl-N-dimethylaminosulfenyl-N-methyl-carbamat mit dem Brechungsindex $n_D^{21,5}$: 1,56o5.

Analog können die Verbindungen der Formel

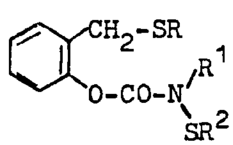

(I)

hergestellt werden:

| Beispiel Nr. | R | $R^1$ | $R^2$ | Brechungsindex: |
|---|---|---|---|---|
| 2 | $C_2H_5$ | $CH_3$ | 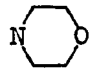 | $n_D^{22}$:1,5630 |
| 3 | $CH_3$ | $CH_3$ | $N(CH_3)_2$ | $n_D^{21}$:1,5639 |
| 4 | n-$C_3H_7$ | $CH_3$ | 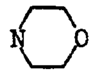 | $n_D^{21}$:1,5618 |
| 5 | iso-$C_3H_7$ | $CH_3$ | 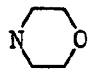 | $n_D^{21}$:1,5592 |
| 6 | $CH_3$ | $CH_3$ | 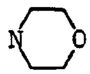 | $n_D^{21}$:1,5733 |
| 7 | iso-$C_3H_7$ | $CH_3$ | $N(CH_3)_2$ | $n_D^{21}$:1,5485 |
| 8 | n-$C_3H_7$ | $CH_3$ | $N(CH_3)_2$ | $n_D^{21}$:1,5487 |
| 9 | $C_2H_5$ | n-$C_4H_9$ | 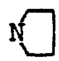 | $n_D^{22}$:1,5448 |
| 10 | $CH_3$ | $CH_3$ | 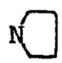 | $n_D^{22}$:1,5754 |
| 11 | iso-$C_3H_7$ | $CH_3$ | 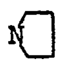 | $n_D^{22}$:1,5642 |
| 12 | $C_2H_5$ | $CH_3$ | 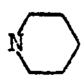 | $n_D^{22}$:1,5601 |
| 13 | n-$C_3H_7$ | $CH_3$ | 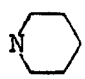 | $n_D^{22}$:1,5552 |
| 14 | $C_2H_5$ | $C_2H_5$ | $N(CH_3)_2$ | $n_D^{26}$:1,5489 |
| 15 | $C_2H_5$ | $C_2H_5$ | 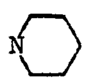 | $n_D^{26}$:1,5576 |

Patentansprüche

1. Aryl-N-alkyl-carbamate der Formel (I)

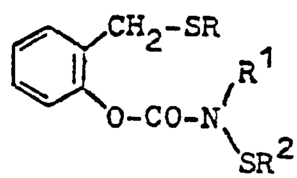

(I)

worin

$R$ und $R^1$ für gleiches oder verschiedenes Alkyl steht und

$R^2$ für Dialkylamino oder einen stickstoffhaltigen Heterocyclus steht, wobei der Heterocyclus noch weitere Heteroatome enthalten kann.

2. Verfahren zur Herstellung der Aryl-N-alkyl-carbamate der Formel (I) gemäß Anspruch 1, dadurch gekennzeichnet, daß man Aryl-N-alkylcarbamate der Formel (II)

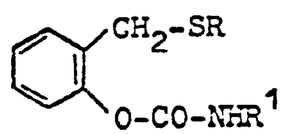

(II)

in welcher

$R$ und $R^1$ die oben angegebene Bedeutung haben,

mit Sulfenylhalogeniden der Formel (III)

$$R^2\text{-S-Hal} \qquad (III)$$

in welcher

$R^2$ die oben angegebene Bedeutung hat und

Hal für Halogen, vorzugsweise Chlor, steht,

gegebenenfalls in Gegenwart eines Säureakzeptors und eines Lösungsmittels umsetzt.

3. Insektizide Mittel gekennzeichnet durch einen Gehalt an Aryl-N-alkyl-carbamate der Formel (I) gemäß Anspruch 1.

4. Verwendung von Aryl-N-alkyl-carbamate der Formel (I) gemäß Anspruch 1 zur Bekämpfung von Insekten.

5. Verfahren zur Bekämpfung von Insekten, dadurch gekennzeichnet, daß man Aryl-N-alkyl-carbamate der Formel (I) gemäß Anspruch 1 auf Insekten und/oder ihren Lebensraum einwirken läßt.

6. Verfahren zur Herstellung insektizider Mittel, dadurch gekennzeichnet, daß man Aryl-N-alkyl-carbamate der Formel (I) gemäß Anspruch 1 mit Streckmitteln und oberflächenaktiven Stoffen vermischt.

Europäisches Patentamt

# EUROPÄISCHER RECHERCHENBERICHT

Nummer der Anmeldung
0000895
EP 78 100 609.3

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | DE - A - 2 655 212 (UPJOHN)<br>* Ansprüche 1, 26, 27 *<br>& FR - A - 2 337 128<br>-- | 1-6 |
| P | DE - A - 2 727 614 (CIBA-GEIGY)<br>* Anspruch 22 *<br>-- | 2 |
| A | DE - A - 2 628 574 (UNION CARBIDE)<br>-- | |
| A | DE - A - 2 632 692 (CIBA-GEIGY)<br>-- | |
| P,A | DE - A - 2 724 764 (CIBA-GEIGY)<br>---- | |

KLASSIFIKATION DER ANMELDUNG (Int.Cl.²)

C 07 C 161/ 00
A 01 N 9/12
C 07 C 149/437
C 07 D 295/22

RECHERCHIERTE SACHGEBIETE (Int. Cl.²)

A 01 N 9/12
C 07 C 149/437
C 07 C 161/00
C 07 D 295/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
| --- | --- | --- |
| Berlin | 14-11-1978 | FROELICH |